# EUROPÄISCHE PATENTANMELDUNG

(11) **EP 4 205 779 A1**
(43) Veröffentlichungstag der Anmeldung: **05.07.2023**
(21) Anmeldenummer: 22216745.4
(22) Anmeldetag: 27.12.2022
(51) Int. Cl.: A61M 5/142, A61M 5/14, A61M 5/168, A61M 39/28

(54) **IV SYSTEMS - INFUSIONSPUMPE ZUR ERKENNUNG DER FARBE EINER SCHLAUCHKLEMME MIT IR-MARKERN**

(30) Priorität: 28.12.2021 DE 102021215067
(71) Anmelder: B. Braun Melsungen AG, 34212 Melsungen (DE)
(72) Erfinder: KATERKAMP, Andreas, 34212 Melsungen (DE); DISCHER, Markus, 34593 Knüllwald (DE); ERLEN, Christoph, 34132 Kassel (DE); BÜRGER, Maria, 34323 Malsfeld (DE); SCHWARZ, Jan, 34212 Melsungen (DE); RICHARDT, Mario, 34289 Zierenberg (DE); HOEVEL, Stephan, 34121 Kassel (DE); FRAGNER, Jan, 34134 Kassel (DE); HERR, Jan Eric, 34119 Kassel (DE); SCHUMACHER, Volker, 88339 Bad Waldsee (DE); PAUL, Simon, 88400 Biberach (DE); JEDELHAUSER, Raphael, 89129 Langenau (DE)
(74) Vertreter: Winter, Brandl - Partnerschaft mbB

(57) **Zusammenfassung**

Die vorliegende Offenbarung betrifft ein Klemmmodul (1) einer oder für eine medizinische Pumpe, welches ein elektro-optisches System zum Erkennen einer Schlauchklemme (2) eines in das Klemmmodul (1) einsetzbaren Infusionseinmalartikels mit folgenden Komponenten aufweist, wobei die Schlauchklemme (2) eine optische Markierung (3) hat: eine erste Lichtquelle (4) zum Senden zumindest eines ersten Lichtstrahls (5), eine zweite Lichtquelle (6) zum Senden zumindest eines zweiten Lichtstrahls (7), wobei die zweite Lichtquelle (6) zur ersten Lichtquelle (4) unterschiedliche ausgebildet ist, einen Photodetektor (8) zum Empfangen eines reflektierten Lichts (9), und ein optisches Fenster (10) zur Bündelung des zumindest ersten Lichtstrahls (5) und/ oder des zumindest zweiten Lichtstrahls (7) auf dem Infusionseinmalartikel (2) sowie des reflektierenden Lichts (9) in dem Photodetektor. Ferner betrifft die vorliegende Offenbarung ein Verfahren zur Erkennung einer Schlauchklemme (2) basierend auf einer Farbe mittels eines Messalgorithmus nach Einschub der Schlauchklemme (2) in eine medizinische Pumpe.

## Beschreibung

### Technisches Gebiet

Die vorliegende Offenbarung betrifft ein Klemmmodul/ Klemmenmodul einer oder für eine medizinische Pumpe, insbesondere Infusionspumpe, welches ein elektro-optisches System zum Erkennen einer Schlauchklemme eines in das Klemmmodul einsetzbaren Infusionseinmalartikels aufweist, und so die Art des Infusionseinmalartikels zu bestimmen. Ferner betrifft die vorliegende Offenbarung ein Verfahren zur Erkennung einer Schlauchklemme basierend auf einer Farbe mittels eines Messalgorithmus nach Einschub der Schlauchklemme in eine medizinische Pumpe.

### Hintergrund der Erfindung

Infusionspumpen und Infusionssets enthalten oft eine Schiebeklemme/ Schlauchklemme. Die Schlauchklemme ist hierbei Teil des Infusionssets und interagiert mit dem Schlauch des Infusionssets, um selektiv einen freien Fluss durch den Infusionsschlauch zu verhindern. Häufig wird eine solche Schlauchklemme durch die Pumpe selbst im Rahmen eines Einsetzvorgangs des Infusionssets in die Pumpe betätigt, etwa mittels einer Frontklappe der Pumpe bei deren Schließbewegung. Alternativ kann die Schlauchklemme aber auch manuell unabhängig von irgendwelchen Schließmechanismen an der Pumpe betätigbar sein.

Die Schlauchklemme weist typischerweise ein sich verjüngendes Langloch auf, durch das der Schlauch verläuft. Der Schlauch ist an einem Ende des Lochs nicht verschlossen und wird zusammengedrückt, wenn er zum anderen Ende des Lochs geschoben wird. In einigen Fällen dient die als Gleitklemme ausgebildete Schlauchklemme funktional als Schlüssel zum Ein- und Ausschalten der Infusionspumpe und kann dabei helfen, den Schlauch des Infusionssets im Pumpenkanal zu befestigen. Bei einigen Pumpen dient die Schlauchklemme/ Schiebeklemme auch als Schlüssel zum Öffnen der Pumpentür, um den Infusionsschlauch zu laden oder zu entladen. Die Pumpentür wird entriegelt, indem ein Teil der Schlauchklemme/ Schiebeklemme in einen Schlitz im Pumpenkörper gedrückt wird. Durch Drücken der Schlauchklemme/ Schiebeklemme in den Schlitz wird der Schlauch zum schmalen Ende des konischen Lochs gedrückt, wodurch der Schlauch geschlossen wird. Somit bietet die Schlauchklemme ein Maß an Sicherheit, indem sie einen freien Fluss verhindert, da sie den Schlauch beim Öffnen der Pumpentür zusammendrückt.

Gleit-/Schiebeklemmen dieser Gattung sind oft passend für eine bestimmte Pumpe und stellen somit sicher, dass nur kompatible Infusionssets mit dieser Pumpe verwendet werden. Vor dem Hintergrund ist es von Bedeutung, sicherstellen zu können, dass die richtige Schlauchklemme (Gleit-/Schiebeklemme) verwendet wird bzw. erkennen zu können, welche Art an Infusionsschlauchset eingesetzt ist.

### Stand der Technik

Aus dem Stand der Technik ist die US 9,272,129 B2 bekannt, in welcher ein Infusionsset bereitgestellt wird, bei dem eine Schiebklemme/ Schlauchklemme durch die Verwendung von Farben, Löchern oder dergleichen kodiert werden kann, um die Art des damit verwendeten Infusionsschlauchs zu identifizieren. Die Infusionspumpe kann den Code auf der Schlauchklemme/ Schiebeklemme erkennen und dadurch den Typ des Infusionsschlauchs bestimmen und dadurch nur den Zugriff auf Infusionsprogramme für Medikamente oder Infusionslösungen ermöglichen, die mit dem jeweiligen Infusionsschlauchtyp kompatibel sind.

Zudem ist aus der US 2006/0224128 A1 ein System und ein Verfahren zum automatischen Verabreichen eines Infusats an einen Patienten offenbart. Das System umfasst ein Infusionsset und ein Infusionsgerät. Eine an einer Infusionsset-Komponente angeordnete Signalisierungskomponente identifiziert ein Verabreichungsprotokoll für das Infusionsset. Eine operativ mit der Infusionsvorrichtung verbundene Erfassungsvorrichtung erfasst die Signalisierungskomponente und identifiziert das Verabreichungsprotokoll. Das Infusionsgerät wird dann so konfiguriert, dass es gemäß dem Verabreichungsprotokoll arbeitet.

Der Stand der Technik bringt jedoch den Nachteil mit sich, dass die Farberkennung der Schlauchklemme unzureichend ist, da Farbspektren bei reiner RGB Beleuchtung nicht ausreichend getrennt werden können.

### Kurzbeschreibung der Erfindung

Vor dem Hintergrund liegt der vorliegenden Offenbarung die objektiv technische Aufgabe zugrunde, die Nachteile aus dem Stand der Technik zu beseitigen oder zumindest zu vermindern, insbesondere eine medizinische Pumpe bereitzustellen, welche ein sicheres Erkennen einer eingelegten Schlauchklemme/ Schiebeklemme ermöglicht.

Daher betrifft die vorliegende Offenbarung ein Klemmmodul einer oder für eine medizinische Pumpe, insbesondere Infusionspumpe, welches ein elektro-optisches System zum Erkennen einer Schlauchklemme eines in das Klemmmodul einsetzbaren Infusionseinmalartikels, insbesondere eines Infusionsschlauchs hat oder ausbildet, wobei die Schlauchklemme eine vorbestimmte Farbe und/oder eine optische/optisch erkennbare Markierung hat. Das Klemmmodul hat eine erste Lichtquelle zum Senden zumindest eines ersten Lichtstrahls, eine zweite Lichtquelle zum Senden zumindest eines zweiten Lichtstrahls, wobei die zweite Lichtquelle zur ersten Lichtquelle unterschiedlich ausgebildet ist, bzw. die Frequenzen/ Wellenlängen der zumindest zwei Lichtstrahlen unterschiedlich zueinander sind, einen Photodetektor zum Empfangen eines reflektierten Lichts, und ein optisches Fenster zur Bündelung des zumindest ersten Lichtstrahls und/ oder des zumindest zweiten Lichtstrahls auf dem Infusionseinmalartikel sowie des reflektierenden Lichts in dem Photodetektor.

In anderen Worten betrifft die vorliegende Erfindung ein Klemmmodul einer medizinischen Pumpe, insbesondere einer Infusionspumpe, mit einem elektro-optischen System zur Erkennung der Farbe/Farbmarkierung einer Schlauchklemme eines Infusionseinmalartikels und zur entsprechenden Steuerung der medizinischen Pumpe/ Infusionspumpe. Dabei wird die Schlauchklemme innerhalb der medizinischen Pumpe/ Infusionspumpe mit unterschiedlichen Lichtquellen/unterschiedlichen Wellenlängen beleuchtet und über das reflektierende Licht, die Farbe der Schlauchklemme ermittelt und auf die Art des Infusionseinmalartikels geschlossen.

Beim Einlegen des Infusionseinmalartikels wird der Infusionseinmalartikel über das Erkennungsmerkmal von einem Sensor und einer Kontrolleinheit in der Infusionspumpe erkannt und zur Aktivierung von Parametern in der Kontrolleinheit der Infusionspumpe genutzt. Diese Parameter sind zum Beispiel
- Parameter zur Steuerung der Druckerkennung,
- Parameter zur Steuerung der Lufterkennung,
- Parameter zur Definition der maximalen Verweildauer des Infusionseinmalartikels und/ oder
- Parameter zu Air-Stop-Filter (ja/nein).

Die zu aktivierenden Parameter können in einem Einmalartikeldatensatz hinterlegt sein. Die Pumpe ermöglicht dem Anwender, die Infusion über die Eingabevorrichtung und das Display zu definieren und zu überwachen. In anderen Worten bedeutet das eine Erweiterung des spektralen Raums bei optisch ähnlichen Farbspektren.

Die vorliegende Offenbarung bringt die Vorteile bzw. Verbesserungen mit sich, dass die Farbe der Schlauchklemme/ Schiebeklemme sowohl für den Nutzer/ Anwender als auch das Gerät nutzbar/ erkennbar ist. Somit bietet die vorliegende Offenbarung gleichzeitig eine Maschinenlesbarkeit und eine Nutzerlesbarkeit. Durch die vorzugsweise zusätzliche optische Markierung lässt sich ein Abbildungsraum erweitern und die Farben entsprechend trennen.

Weitere vorteilhafte Ausführungsformen sind nachfolgend gemäß den Unteransprüchen beschrieben.

Es ist bevorzugt, wenn die erste Lichtquelle eine RGB-LED, vorzugsweise eine kombinierte RGB-LED, ist und die zweite Lichtquelle eine IR-LED, insbesondere mit einer Wellenlänge von 950nm ± 42nm, ist, wobei die erste Lichtquelle drei LEDs aufweist, welche derart ausgebildet sind, um den ersten Lichtstrahl als einen roten, insbesondere einer Wellenlänge von 630nm ± 16nm, einen grünen, insbesondere einer Wellenlänge von 520nm ± 33nm und/ oder einen blauen, insbesondere einer Wellenlänge von 467nm ± 25nm ersten Lichtstrahl auszugeben.

In anderen Worten bedeutet das, dass das elektro-optische System innerhalb der medizinischen Pumpe bzw. innerhalb des in die medizinische Pumpe einsetzbare/eingesetzte Klemmmodul die kombinierte RGB-LED, die IR-LED, den Photodetektor und das optische Fenster zur Bündelung des Lichtstrahls auf der Schlauchklemme sowie des reflektierten Lichts in den Photodetektor hat oder ausbildet. Zur differenzierten Farberkennung werden daher RGB-Signale und IR-Signale zur Beleuchtung der Schlauchklemme genutzt.

Demzufolge werden in der vorliegenden Offenbarung die folgenden Wellenlängen verwendet:
Rot: Hauptwellenlänge 630 nm ± 16 nm
Grün: Hauptwellenlänge 520 nm ± 33 nm
Blau: Hauptwellenlänge 467 nm ± 25 nm
Infrarot (IR): Hauptwellenlänge 950 nm ± 42 nm

Bezüglich der eingesetzten Materialien ergibt sich daraus folgende Übersicht (Lookup-Table) zu den Materialeigenschaften. Diese Anteile sind abhängig von dem Material. Daher ist es naheliegend, dass sich die Anteile bei Einsatz anderer Materialien ändern. In anderen Worten werden in Abhängigkeit des eingesetzten Materials und der Farb-/Pigmentbeimischung der Klemme die eingestrahlten Hauptwellenlängen an der Klemme reflektiert. Diese Reflektion wird in Form einer Look-up Tabelle in der Pumpe hinterlegt, um über einen relativen Vergleich der Einzelfarben (RGB, IR) zueinander, die Farbe der Schlauchklemme über die Look-up Tabelle zu ermitteln.

Dies bedeutet, dass bei Verwendung anderer Materialien in der Pumpe eine neue Lookup-Table eingepflegt werden muss. Es ist jedoch bevorzugt, wenn die sich daraus ergebenden Remissionsspektren in ihren Eigenschaften unverändert bleiben.

| Farbe | Normierte Remission für blau (Hauptwellenlänge 466 nm) | Normierte Remission für grün (Hauptwellenlänge 518 nm) | Normierte Remission für rot (Hauptwellenlänge 629 nm) |
|---|---|---|---|
| Magenta | [13.7 % .. 18.1 %] | [8.3 % .. 10.1 %] | [52.5 % .. 73.0 %] |
| Grün | [64.5 % .. 86.5 %] | [79.3 % .. 100.8 %] | [15.5 % .. 19.5 %] |
| Grau | [108.0 % .. 126.0 %] | [113.5 % .. 133.3 %] | [104.5 % .. 122.6 %] |
| Rot (mit IR Absorber) | [23.3 % .. 28.4 %] | [22.1 % .. 26.4 %] | [170.7 % .. 208.6 %] |
| Weiß | [226.7 % .. 265.8 %] | [231.9 % .. 271.7 %] | [217.2 % .. 257.7 %] |
| Gelb (mit IR Absorber) | [35.1 % .. 52.6 %] | [106.8 % .. 130.8 %] | [113.0 % .. 132.6 %] |
| Pink | [66.0 % .. 84.6 %] | [39.9 % .. 49.6 %] | [55.3 % .. 67.2 %] |
| Blau | *Alternative Option* | *Alternative Option* | *Alternative Option* |
| Orange | *Alternative Option* | *Alternative Option* | *Alternative Option* |

Es ist von Vorteil, wenn die optische Markierung ein Infrarot-Marker ist.

Es ist bevorzugt, wenn das optische Fenster aus Kunststoff ist.

Es ist vorteilhaft, wenn die erste Lichtquelle, die zweite Lichtquelle und der Photodetektor auf einer Leiterplatine angeordnet sind, welche in/ an das Klemmmodul integrierbar/ anbringbar ist.

Es ist bevorzugt, wenn die drei LEDs der ersten Lichtquelle eine lineare Anordnung auf der Leiterplatine haben und in einem so geringen Abstand angeordnet sind, um durch das optische Fenster auszuleuchten, vorzugsweise in einem Abstand von 0,23mm.

In anderen Worten sind die kombinierte RGB-LED und die IR-LED dicht zusammen platziert, damit ihre Ausleuchtung durch das optische Fenster ermöglicht wird. Zudem ist eine RGB-LED verbaut, die es ermöglicht, alle Leuchtquellen in einer Flucht, also gemäß einer linearen Anordnung der Chiplagen, anzuordnen. Der Abstand der RGB-LEDs zueinander beträgt ca. 0,23 mm. Das Layout der Platine und die Auswahl der LEDs ist so gewählt, um möglichst kleine Abmessungen zu haben und um sich in das Klemmenmodul der medizinischen Pumpe/ Infusionspumpe integrieren zu lassen.

Es ist von Vorteil, wenn das optische Fenster zwischen der Schlauchklemme/ Schiebeklemme und der Leiterplatine derart angeordnet ist, dass der zumindest eine erste Lichtstrahl und der zumindest eine zweite Lichtstrahl durch das optische Fenster auf die Schlauchklemme, insbesondere die optische Markierung, trifft und die Schlauchklemme, insbesondere die optische Markierung, den zumindest einen ersten Lichtstrahl und den zumindest einen zweiten Lichtstrahl reflektiert und das reflektierte Licht durch das optische Fenster auf den Photodetektor trifft. Ferner ist es bevorzugt, wenn die Anordnung derart vorgesehen ist, dass das optische Fenster und die Anordnung von Sende-LEDs und Photodetektor auch dafür sorgen, dass keine direkte Lichteinkoppelung zwischen Photodetektor und Sende-LEDs vorhanden ist.

In anderen Worten wird durch die Anordnung der Lichtquellen, des Photodetektors und des zugehörigen optischen Fensters sichergestellt, dass der Reflexionsbereich optimal ausgeleuchtet und dessen Reflexion gebündelt am Photodetektor aufgenommen wird. Das optische Fenster dient daher zum Senden und Empfangen zumindest eines Lichtstrahls und des reflektierten Lichts.

Ferner betrifft die vorliegende Offenbarung eine medizinische Pumpe, insbesondere Infusionspumpe, mit einem Klemmmodul gemäß einem der vorstehenden Aspekte.

Des Weiteren betrifft die vorliegende Offenbarung ein Verfahren zur Erkennung einer Schlauchklemme basierend auf zumindest einer Farbe mittels eines Messalgorithmus nach Einschub der Schlauchklemme in eine medizinische Pumpe, insbesondere Infusionspumpe, um die Art des damit verwendeten Infusionseinmalartikels, insbesondere eines Infusionsschlauchs, zu bestimmen. Ein erster Schritt (S1) ist die Messung einer Temperatur. In einem zweiten Schritt (S2) wird das Umgebungslicht gemessen und die Umgebungshelligkeit ermittelt. Anschließend folgt im dritten Schritt (S3) eine sequentielle Beleuchtung mit rotem, grünen, blauem und IR-Licht und Messung der Reflexion an der Schlauchklemme/ Schiebeklemme. In einem letzten Schritt (S4) erfolgt eine Auswertung und Bestimmung der Farbe durch gemessene Reflexion und anschließendem Abgleich mit einer in der medizinischen Pumpe hinterlegten Auswertetabelle.

In anderen Worten wird nach Einschub der Schlauchklemme folgendes durchgeführt:
- Temperaturmessung;
- Messung des Umgebungslichts (Dunkelkorrektur) und daraus Ermittlung der Umgebungshelligkeit;
- Sequentielle Messung der Reflexion an der Schlauchklemme/ Schiebeklemme, wenn sie nacheinander mit, Rot, Grün, Blau, Infrarotem (IR) Licht beleuchtet wird;
- Die Farbe wird durch Auswertung der gemessenen Informationen (separate Einzelfarbmessung) und anschließendem Abgleich mit einer in der Infusionspumpe abgelegten Auswertetabelle bestimmt. Die Auswertetabelle (Lookup-Table) ermöglicht ausgehend von den Messwerten (Remissionswerten) eine Zuordnung der Farbe.

Es ist bevorzugt, wenn Kalibrierdaten in einem Mikroprozessor vorliegen, welche zur Initialisierung des Messalgorithmus verwendet werden, und die Auswertetabelle eine optische Charakterisierung der eingesetzten Schlauchklemme/ Schiebeklemme bezüglich derer Reflexion aufweist und mittels eines darin hinterlegten Wertebereichs Schwankungen der Messung ausgleichbar sind.

In anderen Worten ist es vorgesehen, dass in die Berechnung der Einzelfarbmessungen die gemessenen Remissionsspektren eingehen. Die Messwerte liegen als sog. "Counts" vor und sind letztlich die Analog-zu-Digital-Messwerte des Photodetektors. Die Messungen sind weiterhin abhängig von den Kalibrierdaten (Weißabgleich), der Umgebungstemperatur und dem Dunkelwert (Umgebungslichteinfluss). Daraus resultiert die nachfolgende Berechnungsgrundlage.

Eine Eingangsvoraussetzung ist das Vorliegen der Kalibrierdaten, vorzugsweise inklusive der Temperaturmessung. Die Kalibrierdaten werden zur "Initialisierung" des Messalgorithmus benötigt. Die Kalibrierdaten erzeugen einen Reflektionskoeffizienten über ein Weißnormal, der in die Berechnung einfließt und in der Gesamtnäherung abhängig von Temperatur und Lichtstärke ist.

Ferner werden die nachfolgenden Messwerte aufgezeichnet. Ein erster Messwert ist die Temperaturmessung (Tmess) und ein zweiter Messwert ist die Beleuchtung der Schlauchklemme nach Einzelfarben (RGB). Der zweite Messwert betrifft Analog/DigitalWerte pro Farbe. In einem weiteren Messwert wird der Dunkelwert aufgezeichnet, welcher einen Messwert beschreibt, der ohne Ausleuchtung der LEDs am Photodetektor anliegt. Es ist ebenfalls nötig, Licht, das von außen in das Klemmmodul eindringen kann, bei der Messung zu kompensieren (entspricht dem vorstehenden Umgebungslicht). Daraufhin erfolgt eine Korrektur des Messsignals bezüglich des Dunkelwerts. Die Messsignale selbst sind die empfangenen Daten an dem Photodetektor zu einem Messzeitpunkt für jede einzelne Farbe. Anschließend folgt eine Temperaturkompensation der Messwerte über ein Polynom, das die Temperaturverläufe der LEDs annähert. Das heißt, die LEDs und deren Lichtstrom verhalten sich für unterschiedlichen Temperaturen unterschiedlich. Daher wird der Verlauf dieser Termperaturdegardation für jede Farbe über eine polynomisch gefittete Temperaturkurve der LED-Farben (Polynom 3. Grades) angenähert. Basierend darauf wird pro Farbe eine Reflektivitätsberechnung durchgeführt, welche auf die Kalibrierdaten normiert ist. Diese Reflektivitätswerte werden mit den in der Lookup-/ Auswertetabelle hinterlegten Minimum- und Maximum-Werten verglichen. Der Vergleich berücksichtigt zusätzliche, mögliche Verschmutzungen, weshalb bei der Farbzuordnung das relative Verhältnis aus Mess- und Auswertetabelle-Wert betrachtet wird. Hierbei können entweder alle Messwerte der vier Farben in die Farbbestimmung eingehen. Alternativ ist auch denkbar, die Intensität/ Amplitude des Skalierungsfaktors zu wählen, wenn es keine eindeutige Farbzuordnung geben sollte.

Die Lookup-Table wird mit der Materialqualifizierung der Schlauchklemme/ Schiebeklemme erzeugt und vermessen. Sie beinhaltet die optische Charakterisierung des eingesetzten Materials bezüglich ihrer Reflexion der Farben rot, grün, blau und IR. Die Auswertetabelle umfasst einen Wertebereich (min./max. Wert), um Schwankungen bei der Farbemessung ausgleichen zu können. Weitere Informationen zu den Wellenlängen sind vorstehend bereits beschrieben worden. Das Einbringen eines IR-Anteils ist nötig, um die Farben Rot und Gelb von anderen Farben mit identischen Reflexionseigenschaften (Remissionsspektrum) unterscheiden zu können. Ohne die eingebrachten IR-Marker, welche homogen in die Schlauchklemme/ Schiebeklemme eingebracht sind, wären die Remissionsspektren zwar in ihrer Amplitude unterschiedlich, jedoch der eigentliche Verlauf und das Verhältnis aller Farben zueinander identisch. Eine eindeutige Erkennung wäre dadurch nicht gegeben.

Zusammenfassend beruht der Messalgorithmus auf der Idee, die Schlauchklemme/ Schiebeklemme innerhalb der Infusionspumpe mit unterschiedlichen Lichtquellen zu beleuchten und über das reflektierende Licht, die Farbe/Farben der Klemme zu ermitteln. Hierzu ist es jedoch mindestens einmalig nötig, eine Kalibrierung des optischen Systems durchzuführen, um die Einflüsse der Einbausituation (hier z.B. Toleranz der Einbaulage) sowie die Toleranzen der elektronischen Bauteile auszugleichen. Durch die Kalibrierung wird ein Offset-Wertepaar generiert, das im Messalgorithmus zur Kompensation verwendet wird.

### Kurzbeschreibung der Figuren

Fig. 1 ist eine Darstellung eines Klemmmoduls mit einer Leiterplatine gemäß der vorliegenden Offenbarung;
Fig. 2 ist eine Darstellung eines Höhenschnitts durch das Klemmmodul wie in Fig. 2 gezeigt gemäß der vorliegenden Offenbarung;
Fig. 3 ist eine Darstellung des Aufbaus einer Leiterplatine gemäß der vorliegenden Offenbarung;
Fig. 4 ist eine Darstellung einer Innenansicht des Klemmmoduls von einer Klemmseite aus gesehen gemäß der vorliegenden Offenbarung;
Fig. 5 ist eine Darstellung einer Außenansicht des Klemmmoduls von der Seite der Leiterplatine aus gesehen gemäß der vorliegenden Offenbarung;
Fig. 6 ist eine schematische Darstellung einer Schlauchklemme; und
Fig. 7 ist eine Darstellung eines Blockschaltbildes einer Farbmessung und deren Umsetzung innerhalb einer Infusionspumpe gemäß der vorliegenden Offenbarung.

### Beschreibung der Ausführungsbeispiele

Nachstehend werden Ausführungsbeispiele der vorliegenden Offenbarung auf der Basis der zugehörigen Figuren beschrieben.

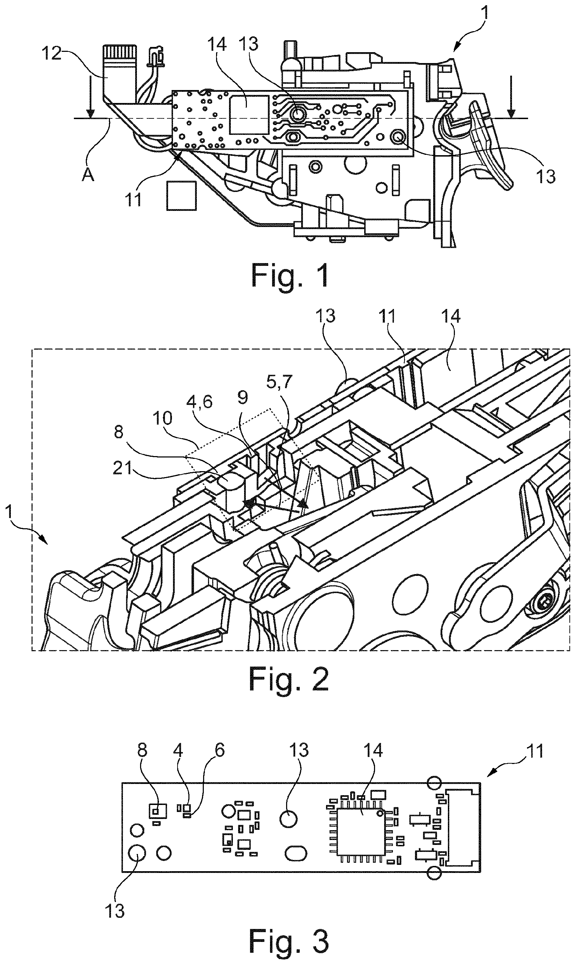

Fig. 1 ist eine Darstellung eines Klemmmoduls 1 mit einer Leiterplatine 11 gemäß der vorliegenden Offenbarung. Das Klemmmodul 1 ist derart ausgebildet, um in eine medizinische Pumpe, insbesondere Infusionspumpe, einsetzbar/ integrierbar zu sein. An dem Klemmmodul 1 ist eine Leiterplatine 11 angebracht bzw. integriert. Die Leiterplatine 11 ist mit deren Oberseite dem Klemmmodul 1 zugewandt. Die Leiterplatine 11 ist mit zumindest einem Befestigungsmittel 13, gemäß Fig. 1 mit zwei Befestigungsmittels 13 an dem Klemmmodul 1 fixiert. Auf der Leiterplatine 11 ist ein erster Teil eines elektro-optisches Systems platziert und ein zweiter Teil des elektro-optischen Systems ist Teil des Klemmmoduls 1. Dies ist in Fign. 2 und 3 detaillierter beschrieben.

Darüber hinaus ist in Fig. 1 ein Flachbandkabel 12 gezeigt, welches auf ein Mainboard/ eine Hauptplatine der medizinischen Pumpe gesteckt wird. Ferner ist auf der Leiterplatine 11 zu erkennen, wo ein Mikroprozessor 14 angeordnet ist. Details hierzu sind zu Fig. 3 näher beschrieben.

Fig. 2 ist eine Darstellung eines Höhenschnitts durch das Klemmmodul 1 wie in Fig. 1 gezeigt gemäß der vorliegenden Offenbarung. In Fig. 1 ist eine Schnittachse A eingezeichnet. Basierend auf der Schnittachse A ist Fig. 2 in einer Draufsicht abgebildet. In Fig. 2 ist daher die Leiterplatine 11 auf der dem Betrachter abgewandten Seite des Klemmmoduls 1 dargestellt. Im Zusammenhang mit der Leiterplatine 11 ist das eine Befestigungsmittel 13 (Schraube, Niete, etc.) zu erkennen, welches dazu dient, die Leiterplatine 11, vorzugsweise mittels Schrauben oder Löten, an dem Klemmmodul 1 zu fixieren.

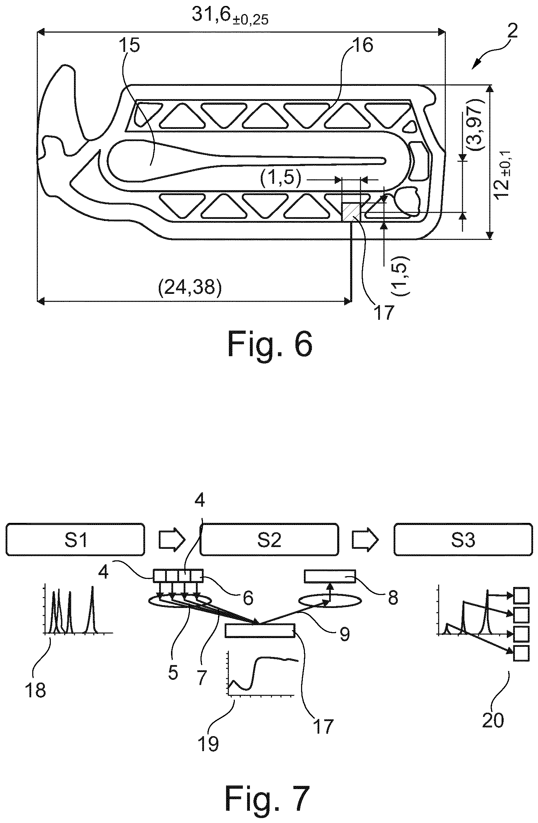

In Fig. 2 ist ferner gezeigt, dass auf der Leiterplatine 11 eine erste Lichtquelle 4, eine zweite Lichtquelle 6 mit entsprechender Sendeoptik und ein Photodetektor 8 mit entsprechender Empfangsoptik angeordnet sind. Zwischen der ersten Lichtquelle 4, der zweiten Lichtquelle 6 und dem Photodetektor 8 ist das optische Fenster 10 angeordnet. Das optische Fenster 10 ist derart ausgebildet, dass zumindest ein erster Lichtstrahl 5 und ein zweiter Lichtstrahl 7 gebündelt durch das optische Fenster 10 auf die einzulegende (hier nicht abgebildete) Schlauchklemme 2 (hier nicht abgebildet, siehe Fig. 6) trifft. Ferner besteht das optische Fenster 10 aus zwei Kunststofflinsen und ist dazu ausgebildet, dass ein von der Schlauchklemme 2 reflektiertes Licht 9 wiederum gebündelt von dem Photodetektor 8 auf der Leiterplatine 11 empfangen wird. Der zumindest eine erste Lichtstrahl 5 und der zweite Lichtstrahl 7 ist in Fig. 2 als ein Pfeil in Richtung weg von der Leiterplatine 11 dargestellt. Das reflektierte Licht 9 ist in Fig. 2 als ein Pfeil in Richtung hin zu der Leiterplatine 11 dargestellt. Das optische Fenster 10 bildet eine derartige Öffnung in dem Gehäuse des Klemmmoduls 1, um die Lichtstrahlen 5 und 7 sowie das reflektierte Licht 9 durchzulassen.

Vor dem Hintergrund ist die Anordnung der Leiterplatine 11, insbesondere in Bezug auf die erste Lichtquelle 4 und die zweite Lichtquelle 6 sowie den Photodetektor 8, zu dem Klemmmodul 1 und der in das Klemmmodul 1 einzulegenden Schlauchklemme klar definiert.

Fig. 3 ist eine Darstellung des Aufbaus einer Leiterplatine 11 gemäß der vorliegenden Offenbarung. Die Leiterplatine 11 ist rückseitig dargestellt. Die Leiterplatine in Fig. 3 zeigt die Anordnung der ersten Lichtquelle 4, der zweiten Lichtquelle 6 und des Photodetektors 8 zueinander. Hierbei wird ersichtlich, dass die erste Lichtquelle 4 und die zweite Lichtquelle 6 nebeneinander in Breitenrichtung der Leiterplatine 11 angeordnet sind. Der Photodetektor 8 ist bezüglich der ersten Lichtquelle 4 und der zweiten Lichtquelle 6 in Längsrichtung hierzu angeordnet.

Ferner zeigt Fig. 3 die beiden Befestigungsmittel 13, sowie einen Mikroprozessor 14, welcher ebenfalls auf der Leiterplatine 11 angeordnet ist.

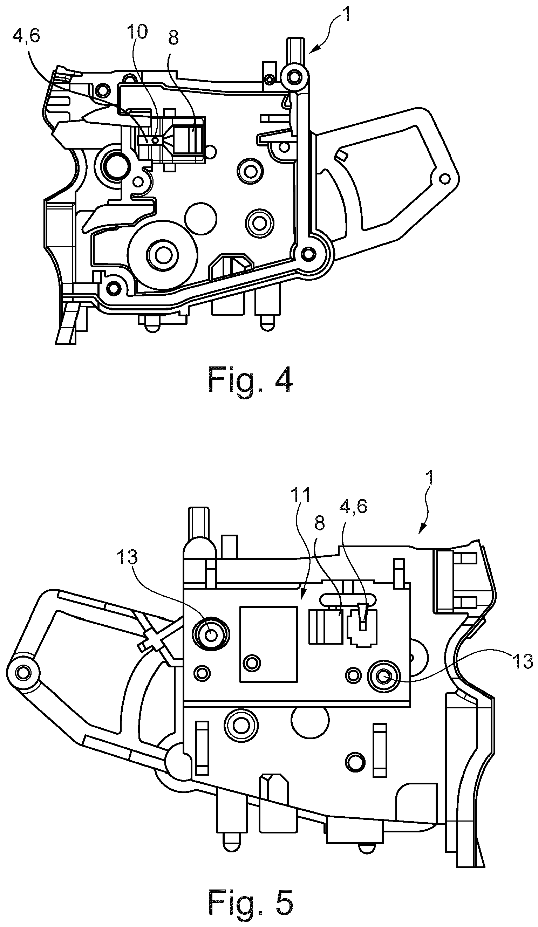

Fig. 4 ist eine Darstellung einer Innenansicht des Klemmmoduls 1 von einer Klemmseite aus gesehen gemäß der vorliegenden Offenbarung. In anderen Worten ist in Fig. 4 ein Schnitt durch das Klemmmodul 1 dargestellt. Hierbei ist die Leiterplatine auf nicht dem Betrachter zugewandten Seite angeordnet und der Schnitt ist derart getätigt, dass das optische Fenster 10 zu sehen ist. Das Klemmmodul 1 zeigt daher das optische Fenster 10, welches aus zwei Kunststofflinsen besteht und hinter welchem der Photodetektor 8 und die erste Lichtquelle 4 sowie die zweite Lichtquelle 6 angeordnet sind.

Fig. 5 ist eine Darstellung einer Außenansicht des Klemmmoduls 1 von der Seite der Leiterplatine 11 aus gesehen gemäß der vorliegenden Offenbarung. In Fig. 5 ist das optische Fenster 10 sowie die "Aufnahme" der Leiterplatine 11 zur Befestigung an dem Klemmmodul 1 schematisch dargestellt. Das heißt, Fig. 5 ist im Gegensatz zu Fig. 4 von der anderen Seite gezeigt. Zudem sind die Befestigungsmittel 13 gezeigt. Auf der dargestellten Außenseite des Klemmmoduls 1 zur Aufnahme der Leiterplatine 11 sind die optischen Fenster 10 bzw. Aussparungen zu erkennen, durch die der Lichtstrahl 5 und 6 der ersten Lichtquelle 4, der zweiten Lichtquelle 6 und des Photodetektors 8 dargestellt. Die Lichtquelle 4 und 6 sowie der Photodetektor 8 sind nicht direkt gezeigt, sondern lediglich deren Position sofern die Leiterplatine 11 in die Aufnahme eingelegt wird.

Fig. 6 ist eine schematische Darstellung einer Schlauchklemme 2. Die Schlauchklemme 2 weist ein sich verjüngendes Langloch 15 auf, durch das ein Infusionsschlauch verläuft. Der Infusionsschlauch ist an einem Ende des Langlochs 15 nicht verschlossen und wird zusammengedrückt, wenn er zum anderen Ende des Langlochs 15 geschoben wird. Beim Einlegen des Einmalartikels ist die Schlauchklemme 2 in einem geöffneten Zustand. In diesem Zustand wird die Schlauchklemme 2 in das Klemmenmodul 1 eingeschoben. Wenn eine Frontklappe der medizinischen Pumpe geschlossen wird, rastet der an der Schlauchklemme 2 gezeigte Haken in einer Öse der Frontklappe ein (und gleichzeitig wird eine pumpenseitige Klemme geschlossen, um den freien Fluss zu verhindern). Beim Öffnen der Frontklappe zieht sich die Schlauchklemme 2 über den Infusionsschlauch und verschließt damit den Durchgang. Parallel öffnet die medizinische Pumpe 1 ihre pumpenseitige Klemme. Beides zusammen verhindert weiterhin den freien Fluss.. Das Bezugszeichen 16 betrifft hierbei lediglich eine Verstärkung der Schlauchklemme 2 selbst. In der Nähe hin zum schmalen Ende des Langlochs 15 weist die Schlauchklemme 2 einen Messpunkt 17 auf. Der Messpunkt 17 ist dazu vorgesehen und ausgebildet, um den zumindest einen ersten Lichtstrahl 5 und den zumindest einen zweiten Lichtstrahl 7 zu reflektieren und das reflektierte Licht 9 an den Photodetektor 8 zurückzusenden.

Ferner sind in Fig. 6 die Größenordnungen des Klemmmoduls 2 angegeben. Die in Fig. 6 angezeigte Gesamtlänge (31 ,6mm) entspricht einer totalen Länge für die Erkennung, dass die Schlauchklemme 2 in das Klemmmodul eingesetzt ist.

Fig. 7 ist eine Darstellung eines Blockschaltbildes einer Farbmessung und deren Umsetzung innerhalb einer Infusionspumpe gemäß der vorliegenden Offenbarung. In einem ersten Schritt S1 erfolgt die sequentielle Beleuchtung der Probe bzw. des Messpunktes 17 der Schlauchklemme 2. Die sequentielle Beleuchtung ergibt Beleuchtungsspektre gemäß der Darstellung zu Bezugszeichen 18. Hierbei trifft der zumindest eine erste Lichtstrahl 5 in den Farben rot, grün und blau ausgehend von der ersten Lichtquelle 4 und der zweite Lichtstrahl 7 ausgehend von zweiten Lichtquelle 6 auf den Messpunkt 17. Der Messpunkt 17 reflektiert in einem Schritt S2 und das reflektierte Licht 9 trifft auf den Photodetektor 8, welcher die spektralen Eigenschaften des Materials gemäß dem Bezugszeichen 19 aufzeichnet. In einem dritten Schritt S3 erzeugt ein Remissionsspektrum einen Fotostrom in dem Photodetektor 8. Das daraus entstehende Signal wird in einem Auswertespektrum gemäß dem Bezugszeichen 20 ausgegeben.

Es ist bevorzugt, wenn thermoplastischer Kunststoff auf Basis von Polyolefin oder Polyoxymethylen mit den folgenden Eigenschaften verwendet werden:
- E-Modul: >1000MPa
- Streckgrenze: >27MPa
- Reibkoeffizienten anhand Stift-Schreibe Reibprüfung in Anlehnung an ASTM G115 und DIN EN ISO 7148-2

| Größe | Wert | |
|---|---|---|
| Aufbau | Prüfstift: Schiebeklemme | |
| | Prüfplatte: Schlauchmaterial | |
| Messung | 20 Zyklen | |
| Zyklus | | 1. Zustellbewegung (tR =2s) |
| | | 2. Stillstand (tP = 2s) |
| | | 3. Rückstellbewegung (tR = 2s) |
| | | 4. Stillstand (tP = 2s) |
| Reibweg | 15mm | |
| Gleitgeschwindigkeit | 450 mm/min | |
| Flächenpressung pN | 2,7 +/- 0,1 MPa | |
| Schlauchmaterial | PVC-P und TPU | |

### Bezugszeichen

- (1): Klemmmodul
- (2): Schlauchklemme
- (3): Markierung
- (4): erste Lichtquelle
- (5): erster Lichtstrahl
- (6): zweite Lichtquelle
- (7): zweiter Lichtstrahl
- (8): Photodetektor
- (9): reflektiertes Licht
- (10): optisches Fenster
- (11): Leiterplatine
- (12): Displaykabel
- (13): Befestigungsmittel
- (14): Mikroprozessor
- (15): Langloch
- (16): Verstärkung
- (17): Messpunkt
- (18): Beleuchtungssprektren
- (19): Spektrale Eigenschaft des Materials
- (20): Auswertespektren

## Patentansprüche

1. Klemmmodul (1) einer oder für eine medizinische Pumpe, insbesondere Infusionspumpe, welches ein elektro-optisches System ausbildet oder hat zum Erkennen einer, zumindest eine vorbestimmte Farbe und/oder eine optische Markierung (3) aufweisenden oder tragenden Schlauchklemme (2) eines in das Klemmmodul (1) einsetzbaren Infusionseinmalartikels, mit folgenden System-Komponenten:
- eine erste Lichtquelle (4) zum Senden zumindest eines ersten Lichtstrahls (5),
- eine zweite Lichtquelle (6) zum Senden zumindest eines zweiten Lichtstrahls (7), wobei die zweite Lichtquelle (6) zur ersten Lichtquelle (4) und/oder die Frequenz des ersten Lichtstrahls zur Frequenz des zweiten Lichtstrahls unterschiedlich ausgebildet ist,
- einen Photodetektor (8) zum Empfangen eines am Infusionseinmalartikel (2), insbesondere an der Schlauchklemme (2) reflektierten Lichts (9), und
- ein optisches Fenster (10) zur Bündelung des zumindest ersten Lichtstrahls (5) und/ oder des zumindest zweiten Lichtstrahls (7) auf dem Infusionseinmalartikel (2), insbesondere auf der Schlauchklemme (2) sowie des reflektierenden Lichts (9) in dem Photodetektor (8).

2. Klemmmodul (1) gemäß Anspruch 1, **dadurch gekennzeichnet, dass** die erste Lichtquelle (4) eine RGB-LED, vorzugsweise eine kombinierte RGB-LED, ist und die zweite Lichtquelle (6) eine IR-LED, insbesondere mit einer Wellenlänge von 950nm ± 42nm, ist, wobei die erste Lichtquelle (4) drei LEDs aufweist, welche derart ausgebildet sind, um den ersten Lichtstrahl (5) als einen roten, insbesondere einer Wellenlänge von 630nm ± 16nm, einen grünen, insbesondere einer Wellenlänge von 520nm ± 33nm und/ oder einen blauen, insbesondere einer Wellenlänge von 467nm ± 25nm ersten Lichtstrahl (5) auszugeben.

3. Klemmmodul (1) gemäß einem der Ansprüche 1 oder 2, **dadurch gekennzeichnet, dass** die optische Markierung (3) ein Infrarot-Marker ist.

4. Klemmmodul (1) gemäß einem der Ansprüche 1 bis 3, **dadurch gekennzeichnet, dass** das optische Fenster (10) aus Kunststoff ist.

5. Klemmmodul (1) gemäß einem der Ansprüche 1 bis 4, **dadurch gekennzeichnet, dass** die erste Lichtquelle (4), die zweite Lichtquelle (6) und der Photodetektor (8) auf einer Leiterplatine (11) angeordnet sind, welche in/ an das Klemmmodul (1) integrierbar/ anbringbar ist.

6. Klemmmodul (1) gemäß Anspruch 5, **dadurch gekennzeichnet, dass** die drei LEDs der ersten Lichtquelle (4) eine lineare Anordnung auf der Leiterplatine (11) haben und in einem so geringen Abstand angeordnet sind, um durch das optische Fenster (10) auszuleuchten, vorzugsweise in einem Abstand von 0,23mm.

7. Klemmmodul (1) gemäß Anspruch 5 oder 6, **dadurch gekennzeichnet, dass** das optische Fenster (10) zwischen der Schlauchklemme (2) und der Leiterplatine (11) derart angeordnet ist, dass der zumindest eine erste Lichtstrahl (5) und der zumindest eine zweite Lichtstrahl (7) durch das optische Fenster (10) auf die Schlauchklemme (2), insbesondere die optische Markierung (3), trifft und die Schlauchklemme (2), insbesondere die optische Markierung (3), den zumindest einen ersten Lichtstrahl (5) und den zumindest einen zweiten Lichtstrahl (7) reflektiert und das reflektierte Licht (9) durch das optische Fenster (10) auf den Photodetektor (8) trifft.

8. Medizinische Pumpe, insbesondere Infusionspumpe, mit einem Klemmmodul (1) gemäß einem der vorstehenden Ansprüche.

9. Verfahren zur Erkennung einer Schlauchklemme (2) basierend auf einer Farbe mittels eines Messalgorithmus nach Einschub der Schlauchklemme (2) in eine medizinische Pumpe, insbesondere Infusionspumpe, mit den folgenden Schritten:
- Messung einer Temperatur;
- Messung des Umgebungslichts und Ermittlung der Umgebungshelligkeit;
- sequentielle Beleuchtung mit rotem, grünen, blauem und IR-Licht und Messung der Reflexion an der Schlauchklemme (2); und
- Auswertung und Bestimmung der Farbe durch gemessene Reflexion und anschließendem Abgleich mit einer in der medizinischen Pumpe hinterlegten Auswertetabelle.

10. Verfahren gemäß Anspruch 9, **dadurch gekennzeichnet, dass** Kalibrierdaten in einem Mikroprozessor vorliegen, welche zur Initialisierung des Messalgorithmus verwendet werden, und die Auswertetabelle eine optische Charakterisierung der eingesetzten Schlauchklemme (2) bezüglich derer Reflexion aufweist und mittels eines darin hinterlegten Wertebereichs Schwankungen der Messung ausgleichbar sind.

11. Schlauchklemme (2) zum Einsetzen in das Klemmmodul (1) gemäß einem der vorhergehenden Ansprüche, **dadurch gekennzeichnet, dass** die vorbestimmte Farbe der Schlauchklemme (2) derart ausgebildet und vorgesehen ist, um von einem Algorithmus nutzbar zu sein und die optische Markierung vorzugsweise dazu vorgesehen und ausgebildet ist, um zusätzlich zur Trennung ähnlicher Farben und/oder Reflexionsspektren zu dienen.
